(19) Europäisches Patentamt
European Patent Office
Office européen des brevets

(11) Numéro de publication : **0 336 812 B1**

(12) **FASCICULE DE BREVET EUROPEEN**

(45) Date de publication du fascicule du brevet : **26.08.92 Bulletin 92/35**

(51) Int. Cl.$^5$ : **A61K 7/06,** A61K 31/62, A61K 31/505

(21) Numéro de dépôt : **89400821.8**

(22) Date de dépôt : **23.03.89**

# (54) Association de dérivés de pyrimidine et de dérivés d'acide salicylique pour induire et stimuler la croissance des cheveux et diminuer leur chute.

Le dossier contient des informations techniques présentées postérieurement au dépôt de la demande et ne figurant pas dans leprésent fascicule.

(30) Priorité : **31.03.88 LU 87187**

(43) Date de publication de la demande : **11.10.89 Bulletin 89/41**

(45) Mention de la délivrance du brevet : **26.08.92 Bulletin 92/35**

(84) Etats contractants désignés : **AT BE CH DE FR GB IT LI NL SE**

(56) Documents cités :
**WO-A-88/07361**
**FR-A- 2 581 542**
**GB-A- 2 175 902**

(56) Documents cités :
**PATENT ABSTRACTS OF JAPAN, vol. 11, no. 115 (C-415)[2562], 10 avril 1987; & JP-A-61 260 010 (YAMAGUCHI YAKUHIN SHOKAIK.K.) 18-11-1986**
**CHEMICAL ABSTRACTS, vol. 78, 1973, page 37, abstract no. 52753c, Columbus, Ohio, US; A. LESPAGNOL et al.: "Analgesic,antiinflammatory and antimicrobial activities of chalcones and dihydrochalcones derived from salicylic acid", & CHIM. THER.1972, 7(5), 365-9**

(73) Titulaire : **L'OREAL**
**14, Rue Royale**
**F-75008 Paris (FR)**

(72) Inventeur : **Rosenbaum, Georges**
**2, rue J.H. Mansart**
**F-92600 Asnières (FR)**
Inventeur : **Hocquaux, Michel**
**70, rue du Rendez-vous**
**F-75012 Paris (FR)**

(74) Mandataire : **Casalonga, Axel et al**
**BUREAU D.A. CASALONGA - JOSSE**
**Morassistrasse 8**
**W-8000 München 5 (DE)**

EP 0 336 812 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

L'invention est relative à l'association de dérivés de pyrimidine et de dérivés d'acide salicylique en vue d'induire et de stimuler la croissance des cheveux et diminuer leur chute.

L'activité des follicules pileux est cyclique. A la phase anagène active, qui dure plusieurs années et au cours de laquelle les cheveux s'allongent, succède une phase de repos (télogène) de quelques mois. A la fin de cette période de repos, les cheveux tombent et un autre cycle recommence.

La chevelure se renouvelle donc en permanence; sur les 100.000 à 150.000 cheveux que comporte une chevelure, à chaque instant 10% environ sont au repos et seront donc remplacés en quelques mois.

Dans presque tous les cas, la chute des cheveux survient chez des sujets prédisposés génétiquement et elle atteint plus particulièrement les hommes. Il s'agit plus particulièrement de l'alopécie androgénétique.

Cette alopécie est une perturbation du renouvellement capillaire gui entraîne, dans un premier temps, une accélération de la fréquence des cycles au dépend de la qualité des cheveux, puis de leur quantité. Il y a un appauvrissement progressif de la chevelure par régression des cheveux dits "terminaux au stade de duvet". Des zones sont touchées préférentiellement : les golfes temporaux, frontaux, entre autres, chez les hommes et on constate une alopécie diffuse du vertex chez les femmes.

On a déjà proposé d'utiliser des composés tels que l'amino-6 dihydro-1,2 hydroxy-1 imino-2 pipéridino-4 pyrimidine ou "Minoxidil" dans des compositions permettant de réduire ou de supprimer l'effet de l'alopécie et d'induire et de stimuler la croissance des cheveux et diminuer leur chute.

On a déjà proposé des compositions pour la croissance des cheveux à base de Minoxidil associé à des agents germicides comme l'acide salicylique ou le méthylsalicylate. Elles sont décrites notamment dans la demande de brevet japonais JP-A-61 260010.

La demanderesse a découvert maintenant, qu'en associant certain dérivés d'acide salicylique avec certains dérivés de pyrimidine, on constatait de façon' surprenante une induction et une stimulation améliorées de la croissance des cheveux et une action sur le freinage de leur chute, ainsi qu'une nette augmentation de la biodisponibilité cutanée et/ou de la pénétration transcutanée.

Les dérivés d'acide salicylique particuliers selon l'invention tels que définis ci-après sont connus en eux-mêmes et sont décrits plus particulièrement dans le brevet français 2 581 542 pour leurs propriétés kératolytiques.

L'association s'est révélée avoir une activité supérieure par rapport aux dérivés de pyrimidine utilisés seuls, les dérivés d'acide salicylique n'ayant en eux-mêmes aucune action sur la croissance capillaire ou le ralentissement de la chute des cheveux.

L'association permet également de constater une action plus rapide au niveau du traitement de la chute des cheveux et permet la mise en oeuvre de dérivés de pyrimidine à des concentrations plus faibles pour une efficacité supérieure ou équivalente.

La demanderesse a découvert par ailleurs que de façon surprenante l'association des dérivés d'acide salicylique avec les dérivés de pyrimidine modifiait de façon importante la solubilité des dérivés de pyrimidine, et présente en particulier l'avantage de permettre une dissolution plus rapide des substances actives constituées par les dérivés de pyrimidine.

Ce phénomène de consolubilisation est particulièrement avantageux dans la mesure où il est réalisé avec un dérivé porteur d'une activité kératolytique et qu'il permet, du fait d'une solubilité plus importante, d'obtenir une meilleure biodisponibilité et/ou pénétration transcutanée.

Afin de déterminer l'efficacité ou la rapidité d'action des compositions de traitement de l'alopécie, on utilise généralement le trichogramme ou le phototrichogramme qui permet de déterminer, entre autre, le pourcentage de cheveux en phase anagène par rapport aux cheveux en phase télogène.

Un objet de l'invention est donc constitué par l'association de dérivés de pyrimidine et de dérivés d'acide salicylique en vue d'induire et de stimuler la croissance des cheveux et diminuer leur chute.

Un autre objet de l'invention est constitué par des compositions cosmétiques et/ou pharmaceutiques contenant ces composés.

L'invention a également pour objet des dispositifs à plusieurs compartiments permettant la mise en oeuvre de l'association conforme à l'invention.

D'autres objets de l'invention apparaîtront à la lecture de la description et des exemples qui suivent.

L'association conforme à l'invention est essentiellement caractérisée par le fait qu'elle comprend :

a) un composant (A) contenant, dans un milieu physiologiquement acceptable, au moins un dérivé de pyrimidine répondant à la formule :

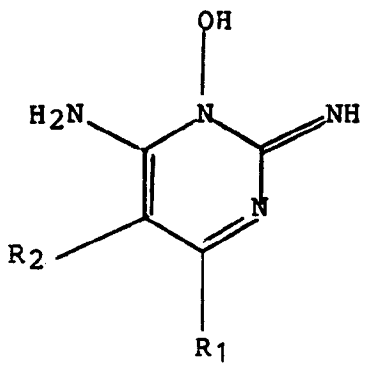

(I)

dans laquelle $R_1$ désigne

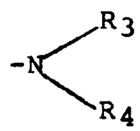

,

$R_3$ et $R_4$, indépendamment l'un de l'autre, désignent hydrogène, un groupement alkyle, alcényle, alkylaryle, cycloalkyle, $R_3$ et $R_4$ pouvant également former un hétérocycle avec l'atome d'azote auquel ils sont liés, choisi parmi les groupements aziridinyle, azétidinyle, pyrrolidinyle, pipéridinyle, hexahydroazépinyle, heptaméthylèneimine, octaméthylèneimine, morpholine et alkyl(inférieur) - 4 pipérazidinyle, les groupements hétérocycliques pouvant être substitués sur les atomes de carbone par un à trois groupements alkyle inférieur, hydroxy ou alcoxy; le groupement $R_2$ est choisi parmi un atome d'hydrogène, un groupement alkyle, alcényle, alkylalcoxy, cycloalkyle, aryle, alkylaryle, arylalkyle, alkylarylalkyle, alcoxyarylalkyle ou haloarylalkyle, ainsi que les sels d'addition d'acides physiologiquement acceptables; et

b) un composant (B), contenant dans un milieu physiologiquement acceptable, au moins un dérivé d'acide salicylique répondant à la formule (II) suivante :

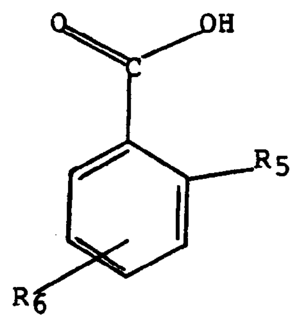

(II)

dans laquelle :

$R_6$ représente un groupement

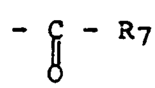

dans lequel $R_7$ désigne une chaîne aliphatique saturée, ayant de 1 à 11 atomes de carbone, linéaire, ramifiée ou cyclisée, une chaîne insaturée ayant de 3 à 17 atomes de carbone, portant une ou plusieurs doubles liaisons conjuguées ou non, ou bien un noyau aromatique lié au radical carbonyle directement ou par l'intermédiaire d'une chaîne aliphatique saturée ou insaturée, ayant de 2 à 7 atomes de carbone, ces différents substituants pouvant eux-mêmes être substitués par un ou plusieurs atomes d'halogène, par un ou plusieurs groupements trifluorométhyle, par un ou plusieurs groupements hydroxyle, le groupement hydroxyle étant présent sous forme libre ou estérifiée par un acide ayant de 1 à 6 atomes de carbone, ou bien par une fonction carboxyle, la fonction carboxyle pouvant être libre ou estérifiée par un alcool inférieur ayant

de 1 à 6 atomes de carbone; $R_6$ représente également un groupement alkyle linéaire ou ramifié, ayant 1 à 18 atomes de carbone ou bien alcoxy comportant 1 à 18 atomes de carbone, linéaire ou ramifié;

$R_5$ représente une fonction hydroxyle ou une fonction ester de formule :

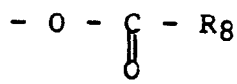

$R_8$ représentant un groupement aliphatique, saturé ou insaturé, ayant de 1 à 15 atomes de carbone,

les composants (A) et (B) faisant partie d'une même composition ou étant destinés à être utilisés séparément, soit simultanément, soit de façon successive ou décalée dans le temps, en vue d'induire et de stimuler la croissance des cheveux et diminuer leur chute.

Pour les composés de formule (I), les groupements alkyle ou alcoxy désignent de préférence un groupement alkyle ou alcoxy inférieur ayant de 1 à 4 atomes de carbone; le groupement alcényle désigne de préférence un groupement alcényle inférieur ayant 2 à 5 atomes de carbone; le groupement aryle désigne de préférence phényle et le groupement cycloalkyle désigne de préférence un groupement ayant 4 à 6 atomes de carbone.

Les composés préférés de formule (I) sont plus particulièrement choisis parmi les composés dans lesquels $R_2$ désigne hydrogène et $R_1$ représente un groupement :

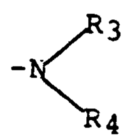

dans lequel $R_3$ et $R_4$ désignent indépendamment l'un de l'autre hydrogène ou un groupement alkyle en $C_1$ à $C_4$ ou $R_3$ et $R_4$ forment un cycle pipéridinyle. On peut citer plus particulièrement le composé constitué par l'amino-6 dihydro-1,2 hydroxy-1 imino-2 diéthylamino-4 pyrimidine et le composé constitué par l'amino-6 dihydro-1,2 hydroxy-1 imino-2 pipéridino-4 pyrimidine encore appelé "Minoxidil". Un sel préféré est plus particulièrement constitué par le sulfate.

Pour les composés de formule (II), le groupement $R_6$ est de préférence en position 5 du cycle et il est de préférence choisi parmi les groupements alcanoyle ayant 2 à 12 atomes de carbone et plus particulièrement parmi les groupements n-octanoyl, diméthyl-3,3 butyroyl, propyl-2 pentanoyl, n-dodécanoyl, n-décanoyl, heptanoyle. Le groupement $R_6$ peut également être choisi parmi les groupements alkyle ayant 1 à 18 atomes de carbone et notamment des groupements hexyle, octyle, décyle. Les groupements alcoxy sont des groupements ayant entre 1 et 18 atomes de carbone et sont plus particulièrement choisis parmi les groupements n-hexyloxy, n-heptyloxy, n-décyloxy.

Les composés particulièrement préférés sont choisis parmi l'acide n-octanoyl-5 salicylique, l'acide n-décanoyl-5 salicylique, l'acide n-dodécanoyl-5 salicylique, l'acide n-octyl-5 salicylique, l'acide n-heptyloxy-5 salicylique, l'acide n-heptyloxy-4 salicylique ou leurs sels ou esters physiologiquement acceptables.

Les dérivés de pyrimidine de formule (I) sont utilisés dans le composant (A) dans des proportions comprises entre 0,05 et 10% en poids et en particulier entre 0,05 et 5% en poids et préférentiellement entre 0,5 et 4% en poids.

Les dérivés d'acide salicylique répondant à la formule (II) sont utilisés dans le composant B dans des proportions comprises entre 0,05 et 10% en poids et en particulier entre 0,05 et 5 % en poids.

Le rapport molaire entre les dérivés de pyrimidine de formule (I) et les dérivés d'acide salicylique de formule (II) est plus particulièrement compris entre 1 et 10.

La demanderesse a constaté que grâce à cette association, l'on pouvant dissoudre une plus grande quantité des dérivés de pyrimidine de formule (I) et plus particulièrement de "Minoxidil", dans le milieu physiologiquement acceptable.

Le milieu physiologiquement acceptable pour les composants (A) et (B) est un milieu utilisable en pharmacie et en cosmétique et peut être constitué par un mélange d'eau et d'un ou plusieurs solvants organiques ou par un mélange de solvants organiques physiologiquement acceptables.

Ces compositions peuvent être pressurisées dans des dispositifs aérosols en présence d'un agent propulseur.

Les solvants plus particulièrement utilisables sont choisis parmi les alcools inférieurs en $C_1$-$C_4$ tels que l'alcool éthylique, l'alcool isopropylique, l'alcool tertiobutylique, les alkylèneglycols comme le propylèneglycol, les alkyl-éthers de mono et de dialkylèneglycol tels que plus particulièrement le monoéthyléther d'éthylèneglycol, le monométhyléther de propylèneglycol et le monoéthyléther de diéthylèneglycol.

Les milieux physiologiquement acceptables peuvent être épaissis ou non. Pour les épaissir, on peut utiliser des agents épaississants et/ou gélifiants bien connus dans l'état de la technique tels que plus particulièrement les hétérobiopolysaccharides tels que la gomme de xanthane ou les scléroglucanes, les dérivés de cellulose, les polymères acryliques réticulés ou non.

Les solvants, lorsqu'ils sont utilisés dans le milieu aqueux, sont présents de préférence dans des proportions comprises entre 1 et 80% en poids par rapport au poids total de la composition.

Les épaississants, lorsqu'ils sont utilisés, sont présents de préférence dans des proportions comprises entre 0,1 et 5% en poids et en particulier entre 0,4 et 3% en poids par rapport au poids total de chacun des composants (A) et (B) lorsque ces composants sont utilisés de façon séparée ou par rapport au poids total de la composition contenant les composants (A) et (B).

Les compositions constituées, soit par l'un et/ou l'autre des composants (A) et (B) ou par la composition contenant les deux composants (A) et (B), peuvent également contenir tous autres adjuvants habituellement utilisés dans les compositions destinées à l'application topique à utilisation cosmétique ou pharmaceutique, et plus particulièrement des agents conservateurs, des agents complexants, des colorants, des agents alcalinisants ou acidifiants, des agents tensio-actifs, anioniques, cationiques, non ioniques, amphotères ou leurs mélanges, des polymères anioniques, cationiques, non ioniques ou amphotères ainsi que leurs mélanges. Le pH de ces compositions peut varier entre 4 et 9.

Une forme de réalisation préférée de l'invention consiste à utiliser l'association sous forme d'une composition contenant les composants (A) et (B), une telle composition constitue un autre objet de l'invention.

Cette forme de réalisation est particulièrement intéressante du fait des propriétés de cosolubilisation du composé de formule (II) sur le composé de formule (I).

Cette composition contient le dérivé de pyrimidine de formule (I) dans des proportions comprises entre 0,05 et 6% en poids par rapport au poids total de la composition et de préférence entre 0,1 et 5% en poids et en particulier entre 0,5 et 2% en poids.

Les dérivés d'acide salicylique de formule (II) sont présents dans des proportions de 0,05 à 10% en poids et de préférence entre 0,05 et 5% en poids.

Une telle composition, du fait des caractéristiques de solubilité améliorées, peut également être préparée tout juste avant l'emploi, lorsque les composants (A) et (B) sont stockés de façon séparée.

Une autre forme de réalisation peut consister à appliquer les composants (A) et (B) de façon séparée, soit simultanément, soit de façon successive ou décalée dans le temps.

Quand on utilise séparément les composants (A) et (B), le composant (A) contient les dérivés de pyrimidine de formule (I), soit sous forme dissoute dans le milieu physiologiquement acceptable ou alors, en totalité ou partiellement en suspension dans ce milieu, en particulier sous forme micronisée, c'est-à-dire sous forme de particules ayant une granulométrie inférieure à 80 microns et de préférence inférieure à 20 microns, et en particulier inférieure à 5 microns.

L'association conforme à l'invention peut être conditionnée dans ce cas dans un dispositif à plusieurs compartiments encore appelé "kit" ou nécessaire, dont un premier compartiment contient le composant (A) renfermant les dérivés de pyrimidine de formule (I) dans un milieu physiologiquement acceptable et le second compartiment contenant le composant (B) à base du dérivé d'acide salicylique de formule (II).

L'association conforme à l'invention, comme indiquée ci-dessus, permet de traiter thérapeutiquement la chute des cheveux en agissant plus particulièrement sur le dysfonctionnement des mécanismes biologiques à l'origine de la pousse des cheveux. Le traitement peut consister à procéder à une application journalière de la composition contenant les composants (A) et (B) définie ci-dessus, pendant une durée de quelques mois, et ce, à raison d'une application par jour.

La demanderesse a constaté que grâce à l'association conforme à l'invention, on améliorait la disponibilité cutanée et la pénétration transcutanée, ce qui augmentait l'efficacité de la composition.

Par ailleurs, la composition présente également l'avantage d'être bactériostatique.

Les compositions conforme à l'invention permettent également de traiter cosmétiquement les cheveux, c'est-à-dire de leur conférer une plus grande vigueur et un aspect meilleur.

L'invention a également pour objet l'utilisation de l'association telle que définie ci-dessus pour la préparation d'un médicament destiné au traitement de l'alopécie agissant notamment sur le dysfonctionnement du cycle pilaire.

Les exemples suivants sont destinés à illustrer l'invention sans pour autant présenter un caractère limitatif.

EXEMPLE 1

On prépare un gel de composition suivante :

| | | | |
|---|---|---|---|
| - Minoxidil | | 3 | g |
| - Acide n-octanoyl-5 salicylique | | 0,55 | g |
| - Gomme de xanthane vendue sous la dénomination KELTROL T par la société KELCO | | 0,75 | g |
| - Alcool éthylique | | 50 | g |
| - Eau | qsp | 100 | g |

Cette composition est appliquée sur les parties alopéciques du cuir chevelu.

EXEMPLE 2

On prépare un gel de composition suivante :

| | | | |
|---|---|---|---|
| - Minoxidil | | 3,2 | g |
| - Acide n-octanoyl-5 salicylique | | 2,25 | g |
| - Hydroxypropyl cellulose vendue sous la dénomination KLUCEL G par la société HERCULES | | 2 | g |
| - Alcool éthylique/propylèneglycol (95/5) | qsp | 100 | g |

Cette composition est appliquée sur les parties alopéciques du cuir chevelu.

EXEMPLE 3

On prépare deux compositions (A) et (B) conditionnées en kit et renfermant respectivement :

Composition (A) :

| | | | |
|---|---|---|---|
| - Minoxidil | | 1,5 | g |
| - Propylène glycol | | 20 | g |
| - Alcool éthylique | | 50 | g |
| - Eau | qsp | 100 | g |

Composition (B) :

| | | | |
|---|---|---|---|
| - Acide n-décanoyl-5 salicylique | | 0,4 | g |
| - Propylène glycol | | 20 | g |
| - Alcool éthylique | | 50 | g |
| - Eau | qsp | 100 | g |

On applique de façon décalée dans le temps la composition (A) le matin et le composition (B) le soir ou vice-versa sur les parties alopéciques du cuir chevelu.

On peut également appliquer les compositions (A) et (B) en alternance journalière (1 jour sur 2 : (A), 1 jour sur 2 : (B)).

EXEMPLE 4

On prépare deux compositions (A) et (B) conditionnées en kit et renfermant respectivement :

Composition (A) :

| | | | |
|---|---|---|---|
| - Minoxidil | | 2 | g |
| - Propylène glycol | | 20 | g |
| - Alcool éthylique | | 50 | g |
| - Eau | qsp | 100 | g |

Composition (B) :

| | | | |
|---|---|---|---|
| - Acide n-dodécanoyl-5 salicylique | | 1,5 | g |
| - Alcool éthylique/propylène glycol (95/5) | qsp | 100 | g |

On applique les compositions (A) et (B) de façon décalée dans le temps : (A) ou (B) le matin, (B) ou (A) le soir ou successivement l'une après l'autre sur les parties alopéciques du cuir chevelu.

EXEMPLE 5

| | | | |
|---|---|---|---|
| - Amino-6 dihydro-1,2 hydroxy-1 imino-2 diéthylamino-4 pyrimidine | | 3 | g |
| - Acide n-octanoyl-5 salicylique | | 2 | g |
| - Propylène glycol | | 20 | g |
| - Alcool éthylique | | 50 | g |
| - Eau | qsp | 100 | g |

Cette composition est appliquée sur les parties alopéciques du cuir chevelu.

EXEMPLE 6

On prépare la lotion suivante :

| | | | |
|---|---|---|---|
| - Minoxidil | | 0,54 | g |
| - Acide n-octanoyl-5 salicylique | | 2,2 | g |
| - Propylène glycol | | 22,8 | g |
| - Alcool éthylique | | 55,1 | g |
| - Eau | qsp | 100 | g |

que l'on applique sur les zones alopéciques du cuir chevelu pour favoriser la repousse et diminuer la chute des cheveux.

EXEMPLE 7

On applique la lotion suivante sur les zones alopéciques du cuir chevelu :

| | | | |
|---|---|---|---|
| - Minoxidil | | 5,0 | g |
| - Acide n-octanoyl-5 salicylique | | 1,09 | g |
| - Alcool éthylique (50g) eau (50g) | qsp | 100,0 | g |

## Revendications

**Revendications pour les Etats contractants suivant : AT, BE, CH, DE, FR, GB, IT, LI, NS, SE**

**1.** Association destinée à induire et à stimuler la croissance des cheveux et diminuer leur chute, caractérisée par le fait qu'elle comprend :

a) un composant (A) contenant dans un milieu physiologiquement acceptable, au moins un dérivé de pyrimidine répondant à la formule :

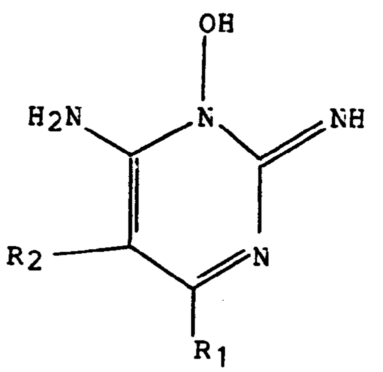

(I)

dans laquelle $R_1$ désigne un groupement

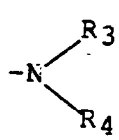

,

dans lequel $R_3$ et $R_4$, indépendamment l'un de l'autre, désignent hydrogène, un groupement alkyle, alcényle, alkylaryle, cycloalkyle, $R_3$ et $R_4$ peuvent également former un hétérocycle avec l'atome d'azote auquel ils sont liés, choisi parmi les groupements aziridinyle, azétidinyle, pyrrolidinyle, pipéridinyle, hexahydroazépinyle, heptaméthylèneimine, octaméthylèneimine, morpholine et alkyl(inférieur)-4 pipérazidinyle, les groupements hétérocycliques pouvant être substitués sur les atomes de carbone par un à trois groupements alkyle inférieur, hydroxy ou alcoxy; le groupement $R_2$ est choisi parmi un atome d'hydrogène, un groupement alkyle, alcényle, alcoxyalkyl, cycloalkyle, aryle, alkylaryle, arylalkyle, alkylarylalkyle, alcoxyarylalkyle ou haloarylalkyle, ainsi que les sels d'addition d'acides physiologiquement acceptables; et

b) un composant (B), contenant dans un milieu physiologiquement acceptable, au moins un dérivé d'acide salicylique répondant à la formule (II) :

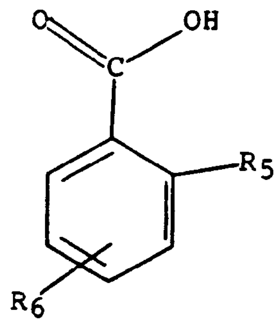

(II)

dans laquelle $R_6$ représente un groupement de formule :

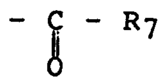

dans laquelle $R_7$ représente une chaîne aliphatique, saturée, ayant de 1 à 11 atomes de carbone, linéaire, ramifiée ou cyclisée, une chaîne insaturée ayant de 3 à 17 atomes de carbone, portant une ou plusieurs doubles liaisons conjuguées ou non, ou bien un noyau aromatique lié au radical carbonyle, directement ou par l'intermédiaire d'une chaîne aliphatique, saturée ou insaturée, ayant de 2 à 7 atomes de carbone, ces différents substituants pouvant être eux-mêmes substitués par un ou plusieurs atomes d'halogène, par un ou plusieurs groupements trifluorométhyle, par un ou plusieurs groupements hydroxyle, sous forme libre ou estérifiée par un acide ayant de 1 à 6 atomes de carbone ou bien par une fonction carboxyle libre ou estérifiée par un alcool inférieur ayant de 1 à 6 atomes de carbone; $R_6$ désigne également un radical alkyle ayant 1 à 18 atomes de carbone, linéaire ou ramifié, ou bien un radical alcoxy ayant 1 à 18 atomes de carbone, linéaire ou ramifié;
$R_5$ représente une fonction hydroxyle ou bien une fonction ester de formule :

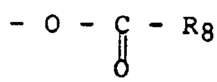

dans laquelle $R_8$ désigne un radical aliphatique, saturé ou insaturé, comportant de 1 à 15 atomes de carbone,

les composants (A) et (B) faisant partie d'une même composition ou étant destinés à être utilisés séparément, soit simultanément, soit de façon successive ou décalée dans le temps, sur les cheveux et le cuir chevelu.

**2.** Association selon la revendication 1, caractérisée par le fait que le composé de formule I est constitué par l'amino-6 dihydro-1,2 hydroxy-1 imino-2 diéthylamino-4 pyrimidine.

**3.** Association selon la revendication 1, caractérisée par le fait que le composé de formule (I) est constitué par l'amino-6 dihydro-1,2 hydroxy-1 imino-2 pipéridino-4 pyrimidine ou "minoxidil".

**4.** Association selon l'une quelconque des revendications 1 à 3, caractérisée par le fait que le dérivé d'acide salicylique est choisi parmi l'acide n-octanoyl-5 salicylique, l'acide n-décanoyl-5 salicylique, l'acide n-dodécanoyl-5 salicylique, l'acide n-octyl-5 salicylique, l'acide n-heptyloxy-5 salicylique, l'acide n-heptyloxy-4 salicylique ou leurs sels ou esters physiologiquement acceptables.

**5.** Association selon l'une quelconque des revendications 1 à 4, caractérisée par le fait que le dérivé de pyrimidine de formule (I) est utilisé dans le composant (A) dans des proportions comprises entre 0,05 et 10% en poids et de préférence compris entre 0,05 et 5% en poids et que le dérivé d'acide salicylique de formule (II) est présent dans le composant (B) dans des proportions comprises entre 0,05 et 10% en poids et de préférence entre 0,05 et 5% en poids.

6. Association selon l'une quelconque des revendications 1 à 5, caractérisée par le fait que le rapport en poids entre le dérivé de pyrimidine de formule (I) et le dérivé d'acide salicylique de formule (II) est compris entre 1 et 10.

7. Association selon l'une quelconque des revendications 1 à 6, caractérisée par le fait que le milieu physiologiquement acceptable est constitué par un mélange d'eau et d'un ou plusieurs solvants organiques ou par un mélange de solvants organiques pharmaceutiquement ou cosmétiquement acceptables.

8. Association selon la revendication 7, caractérisée par le fait que les solvents sont choisis parmi les alcools inférieurs en $C_1$-$C_4$, les alkylèneglycols, les alkyléthers de mono et de dialkylèneglycol.

9. Association selon l'une quelconque des revendications 1 à 8, caractérisée par le fait que l'un au moins des milieux physiologiquement acceptables des composants (A) et (B) est épaissi au moyens d'agents épaississants et/ou gélifiants.

10. Association selon l'une quelconque des revendications 1 à 9, caractérisée par le fait que l'un au moins des composants (A) et/ou (B) contient également au moins un adjuvant cosmétiquement ou pharmaceutiquement acceptable choisi parmi des agents conservateurs, des agents complexants, des colorants, des agents alcalinisants ou acidifiants, des agents tensio-actifs, anioniques, non ioniques ou amphotères ainsi que leurs mélanges, des polymères anioniques, cationiques, non ioniques ou amphotères ainsi que leurs mélanges.

11. Association selon l'une quelconque des revendications 1 à 10, caractérisée par le fait qu'elle se présente sous forme d'une composition unique contenant les composants (A) et (B).

12. Dispositif à plusieurs compartiments ou "kit" ou nécessaire, caractérisé par le fait qu'il comprend dans un premier compartiment le composant (A) contenant dans un milieu physiologiquement acceptable, au moins un dérivé de pyrimidine répondant à la formule (I) et dans un second compartiment, le composant (B) contenant dans un milieu physiologiquement acceptable, au moins un dérivé d'acide salicylique de formule (II).

13. Association selon l'une quelconque des revendications 1 à 11 pour son application comme médicament dans le traitement de l'alopécie.

14. Utilisation de l'association selon l'une quelconque des revendications 1 à 11 pour la préparation d'un médicament destiné à traiter l'alopécie.

15. Procédé de traitement cosmétique des cheveux ou du cuir chevelu comprenant l'application de l'association définie dans l'une quelconque des revendications 1 à 11.

## Patentansprüche

### Patentansprüche für folgenden Vertragsstaat : AT, BE, CH, DE, FR, GB, IT, LI, NS, SE

1. Assoziat zum Induzieren und Stimulieren des Haarwuchses und zum Vermindern des Haarausfalles, dadurch **gekennzeichnet**, daß
es umfaßt:
a) eine Komponente (A), enthaltend in einem physiologisch verträglichen Milieu mindestens ein Pyrimidinderivat der Formel (I):

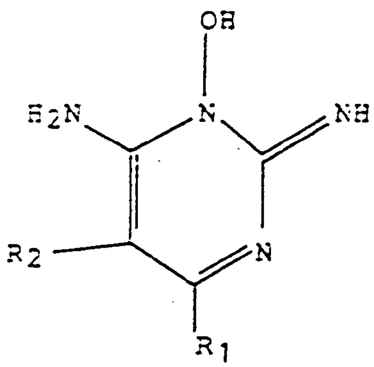

(I)

worin $R_1$ eine

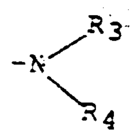

-Gruppe darstellt, worin $R_3$ und $R_4$, unabhängig voneinander, Wasserstoff, eine Alkyl-, Alkenyl-, Alkylaryl-, Cycloalkylgruppe darstellen, wobei $R_3$ und $R_4$ auch einen Heterozyklus mit dem Stickstoffatom, an das sie gebunden sind, bilden können, ausgewählt aus Aziridinyl-, Azetidinyl-, Pyrrolidinyl-, Piperidinyl-, Hexahydroazepinyl-, Heptamethylenimin-, Octamethylenimin-, Morpholinund Niedrigalkyl-4-piperazidinylgruppen, wobei die heterozyklischen Gruppen an den Kohlenstoffatomen durch eine bis drei Niedigalkyl-, Hydroxy- oder Alkoxygruppen substituiert sein können, und worin die $R_2$-Gruppe unter Wasserstoff, einer Alkyl-, Alkenyl-, Alkoxyalkyl-, Cycloalkyl-, Aryl-, Alkylaryl-, Arylakyl-, Alkylarylalkyl-, Alkoxyarylalkyl- oder Haloarylalkylgruppe ausgewählt ist, sowie die physiologisch verträglichen Säureadditionssalze davon; und

b) eine Komponente (B), enthaltend in einem physiologisch verträglichen Milieu mindestens ein Salicylsäurederivat der Formel (II):

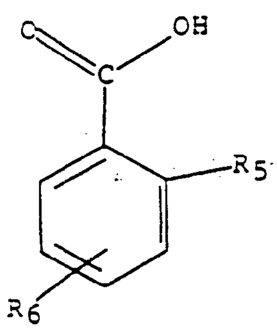

(II)

worin $R_6$ eine Gruppe der Formel darstellt:

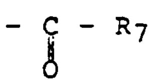

worin $R_7$ eine gesättigte aliphatische Kette mit 1 bis 11 Kohlenstoffatomen, linear, verzweigt oder zyklisch, eine ungesättigte Kette mit 3 bis 17 Kohlenstoffatomen mit einer oder mehreren Doppelbindungen, konjugiert oder nicht, oder auch einen aromatischen Kern darstellt, der an den Carbonylrest gebunden ist, direkt oder über eine dazwischenliegende aliphatische Kette, gesättigt oder ungesättigt, mit 2 bis 7 Kohlenstoffatomen, wobei die verschiedenen Substituenten ihrerseits durch ein oder mehrere Halogenatome, eine oder mehrere Trifluormethylgruppen, eine oder mehrere Hydroxylgruppen, in freier Form oder verestert mit einer Säure mit 1 bis 6 Kohlenstoffatomen, oder auch durch eine freie

oder mit einem Niedrigalkohol mit 1 bis 6 Kohlenstoffatomen veresterte Carboxylfunktion substituiert sein können; worin $R_6$ auch einen linearen oder verzweigten Alkylrest mit 1 bis 18 Kohlenstoffatomen oder auch einen linearen oder verzweigten Alkoxyrest mit 1 bis 18 Kohlenstoffatomen darstellt; und worin
$R_5$ eine Hydroxylfunktion oder auch eine Esterfunktion der Formel darstellt:

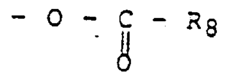

worin $R_6$ einen gesättigten oder ungesättigten aliphatischen Rest mit 1 bis 15 Kohlenstoffatomen darstellt,
wobei die Komponenten (A) und (B) als Bestandteil ein und derselben Zusammensetzung vorliegen oder dazu bestimmt sind, auf den Haaren oder behaarter Haut getrennt verwendet zu werden, entweder gleichzeitig oder aufeinanderfolgend oder nach einem Zeitablauf.

2. Assoziat gemäß Anspruch 1,
dadurch **gekennzeichnet**, daß
die Verbindung der Formel I Amino-6-dihydro-1,2-hydroxy-1-imino-2-diethylamino-4-pyrimidin ist.

3. Assoziat gemäß Anspruch 1,
dadurch **gekennzeichnet**, daß
die Verbindung der Formel I Amino-6-dihydro-1,2-hydroxy-1-imino-2-piperidino-4-pyrimidin oder "Minoxidil" ist.

4. Assoziat gemäß eines jeden der Ansprüche 1 bis 3,
dadurch **gekennzeichnet**, daß
das Salicylsäurederivat aus n-Octanoyl-5-, n-Decanoyl-5-, n-Dodecanoyl-5-, n-Octyl-5-, n-Heptyloxy-5-, n-Heptyloxy-4-salicylsäure oder ihren physiologisch verträglichen Salzen oder Estern ausgewählt ist.

5. Assoziat gemäß eines jeden der Ansprüche 1 bis 4,
dadurch **gekennzeichnet**, daß
das Pyrimidinderivat der Formel (I) in der Komponente (A) in Mengen von 0,05 bis 10, vorzugsweise von 0,05 bis 5, Gewichtsprozent und das Salicylsäurederivat der Formel (II) in der Komponente (B) in Mengen von 0,05 bis 10, vorzugsweise von 0,05 bis 5, Gewichtsprozent verwendet werden.

6. Assoziat gemäß eines jeden der Ansprüche 1 bis 5,
dadurch **gekennzeichnet**, daß
das Gewichtsverhältnis zwischen dem Pyrimidinderivat der Formel (I) und dem Salicylsäurederivat der Formel (II) im Bereich von 1 bis 10 liegt.

7. Assoziat gemäß eines jeden der Ansprüche 1 bis 6,
dadurch **gekennzeichnet**, daß
das physiologisch verträgliche Milieu aus einer Mischung aus Wasser und einem oder mehreren organischen Lösungsmitteln oder aus einer Mischung aus pharmazeutisch oder kosmetisch verträglichen organischen Lösungsmitteln zusammengesetzt ist.

8. Assoziat gemäß Anspruch 7, dadurch **gekennzeichnet**, daß
die Lösungsmittel aus $C_1$-$C_4$-Niedrigalkoholen, Alkylenglycolen und Alkylethern von Mono- und Dialkylenglycol ausgewählt sind.

9. Assoziat gemäß eines jeden der Ansprüche 1 bis 8,
dadurch **gekennzeichnet**, daß
mindestens eines der physiologisch verträglichen Medien der Komponenten (A) und (B) durch Verdickungsmittel und/oder Geliermittel verdickt ist.

10. Assoziat gemäß eines jeden der Ansprüche 1 bis 9,
dadurch **gekennzeichnet**, daß

mindestens eine der Komponenten (A) und/oder (B) ebenfalls mindestens einen kosmetisch oder pharmazeutisch verträglichen Hilfsstoff enthält, ausgewählt aus Konservierungsmitteln, Komplexiermitteln, Färbemitteln, alkalisch oder sauer machenden Mitteln, anionischen, kationischen, nicht-ionischen oder amphoteren oberflächenaktiven Mitteln und aus anionischen, kationischen, nicht-ionischen oder amphoteren Polymeren sowie deren Mischungen.

**11.** Assoziat gemäß eines jeden der Ansprüche 1 bis 10,
dadurch **gekennzeichnet**, daß
es in Form einer einzigen Zusammensetzung vorliegt, enthaltend die Komponenten (A) und (B).

**12.** Vorrichtung aus mehreren Bestandteilen oder "Kit" oder Necessaire,
dadurch **gekennzeichnet**, daß
sie in einem ersten Bestandteil die Komponente (A), enthaltend in einem physiologisch verträglichen Milieu mindestens ein Pyrimidinderivat der Formel (I), und in einem zweiten Bestandteil die Komponente (B), enthaltend in einem physiologisch verträglichen Milieu mindestens ein Salicylsäurederivat der Formel (II), umfassen.

**13.** Assoziat gemäß eines jeden der Ansprüche 1 bis 11 zur Verwendung als Medikament bei der Behandlung der Alopezie.

**14.** Verwendung des Assoziats gemäß eines jeden der Ansprüche 1 bis 11 zur Herstellung eines Medikaments zur Behandlung der Alopezie.

**15.** Verfahren zur kosmetischen Behandlung der Haare oder von behaarter Haut, wobei man das Assoziat gemäß eines jeden der Ansprüche 1 bis 11 aufträgt.

## Claims

### Claims for the following Contracting States : AT, BE, CH, DE, FR, GB, IT, LI, NS, SE

**1.** Combination for inducing and stimulating hair growth and reducing its loss, characterised in that it comprises:

a) a component (A) containing in a physiologically acceptable medium at least one pyrimidine derivative of the formula:

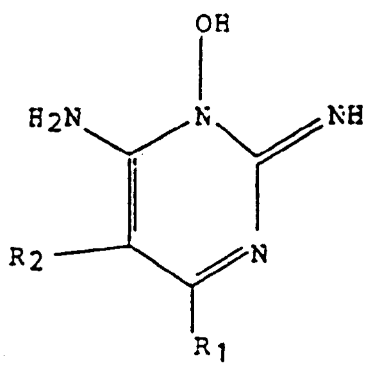

(I)

in which $R_1$ denotes a group

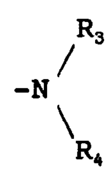

,

in which $R_3$ and $R_4$, independently of each other, denote hydrogen, an alkyl, alkenyl, alkylaryl or cycloalkyl group, $R_3$ and $R_4$ can also form a heterocycle with the nitrogen atom to which they are attached, chosen from the following groups, aziridinyl, azetidinyl, pyrrolidinyl, piperidinyl, hexahydroazepinyl, heptamethyleneimine, octamethyleneimine, morpholine and 4-(lower alkyl)piperazidinyl, it being possible for the heterocyclic groups to be substituted on the carbon atoms by one to three lower alkyl, hydroxy or alkoxy groups; the $R_2$ group is chosen from a hydrogen atom, an alkyl, alkenyl, alkoxyalkyl, cycloalkyl, aryl, alkylaryl, arylalkyl, alkylarylalkyl, alkoxyarylalkyl or haloarylalkyl group, as well as the addition salts of physiologically acceptable acids; and

b) a component (B) containing in a physiologically acceptable medium at least one salicylic acid derivative of the formula (II):

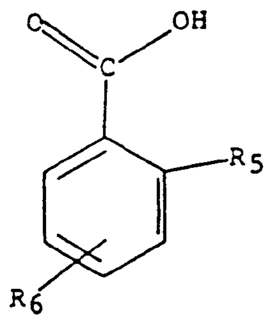

(II)

in which $R_6$ represents a group of the formula:

$$-\underset{\underset{O}{\|}}{C}-R_7$$

in which $R_7$ represents a saturated aliphatic chain having 1 to 11 linear, branched or cyclic carbon atoms, an unsaturated chain having 3 to 17 carbon atoms, bearing one or more conjugated or unconjugated double bonds, or alternatively an aromatic ring attached to the carbonyl radical, directly or through a saturated or unsaturated aliphatic chain having 2 to 7 carbon atoms, it being possible for these different substituents themselves to be substituted by one or more halogen atoms, by one or more trifluoromethyl groups, by one or more hydroxyl groups in free form or esterified by an acid having 1 to 6 carbon atoms, or alternatively by a carboxyl functional group which is free or esterified by a lower alcohol having 1 to 6 carbon atoms; $R_6$ also denotes a linear or branched alkyl radical having 1 to 18 carbon atoms or, alternatively, a linear or branched alkoxy radical having 1 to 18 carbon atoms;

$R_5$ represents a hydroxyl functional group or, alternatively, an ester functional group of the formula:

$$-O-\underset{\underset{O}{\|}}{C}-R_8$$

in which $R_6$ denotes a saturated or unsaturated aliphatic radical comprising 1 to 15 carbon atoms,

the components (A) and (B) being part of the same composition or being intended to be used separately on the hair and the scalp either simultaneously or successively or spaced out over time.

2. Combination according to Claim 1, characterised in that the compound of formula I consists of 6-amino-1,2-dihydro-1-hydroxy-2-imino-4-diethylaminopyrimidine.

3. Combination according to Claim 1, characterised in that the compound of formula (I) consists of 6-amino-1,2-dihydro-1-hydroxy-2-imino-4-piperidinopyrimidine or "minoxidil".

4. Combination according to any one of Claims 1 to 3, characterised in that the salicylic acid derivative is chosen from 5-n-octanoylsalicylic acid, 5-n-decanoylsalicylic acid, 5-n-dodecanoylsalicylic acid, 5-n-octylsalicylic acid, 5-n-heptyloxysalicylic acid, 4-n-heptyloxysalicylic acid or their physiologically acceptable salts or esters.

5. Combination according to any one of Claims 1 to 4, characterised in that the pyrimidine derivative of formula (I) is used in the component (A) in proportions of between 0.05 and 10% by weight and preferably between 0.05 and 5% by weight and in that the salicylic acid derivative of formula (II) is present in the component (B) in proportions of between 0.05 and 10% by weight and preferably between 0.05 and 5% by weight.

6. Combination according to any one of Claims 1 to 5, characterised in that the weight ratio of the pyrimidine derivative of formula (I) to the salicylic acid derivative of formula (II) is between 1 and 10.

7. Combination according to any one of Claims 1 to 6, characterised in that the physiologically acceptable medium consists of a mixture of water and one or more organic solvents or of a mixture of pharmaceutically or cosmetically acceptable organic solvents.

8. Combination according to Claim 7, characterised in that the solvents are chosen from $C_1$-$C_4$ lower alcohols, alkylene glycols, mono- and dialkylene glycol alkyl ethers.

9. Combination according to any one of Claims 1 to 8, characterised in that at least one of the physiologically acceptable media of the components (A) and (B) is thickened by means of thickening and/or gelling agents.

10. Combination according to any one of Claims 1 to 9, characterised in that at least one of the components (A) and/or (B) also contains at least one cosmetically or pharmaceutically acceptable adjuvant chosen from preserving agents, complexing agents, dyes, alkalinising or acidifying agents, anionic, nonionic or amphoteric surface-active agents as well as their mixtures, anionic, cationic, nonionic or amphoteric polymers as well as their mixtures.

11. Combination according to any one of Claims 1 to 10, characterised in that it is provided in the form of a single composition containing the components (A) and (B).

12. Multicompartment device or "kit" or box, characterised in that it comprises in a first compartment, the component (A) containing in a physiologically acceptable medium, at least one pyrimidine derivative of the formula (I) and in a second compartment, the component (B) containing in a physiologically acceptable medium, at least one salicylic acid derivative of formula (II).

13. Combination according to any one of Claims 1 to 11 to be applied as medicinal product in the treatment of alopecia.

14. Use of the combination according to any one of Claims 1 to 11 for the preparation of a medicinal product for treating alopecia.

15. Process of cosmetic treatment of the hair or the scalp, comprising the application of the combination defined in any one of Claims 1 to 11.